# EUROPEAN PATENT APPLICATION

(11) **EP 1 192 939 A2**
(43) Date of publication of application: **03.04.2002**
(21) Application number: 01308359.7
(22) Date of filing: 01.10.2001
(51) Int. Cl.: A61K 7/48, A61K 31/13, A61K 31/195

(54) **Methods for reduction of inflammation and erythema**

(30) Priority: 02.10.2000 US 677737
(71) Applicant: Johnson & Johnson Consumer Companies, Inc., Skillman, New Jersey 08558-9418 (US)
(72) Inventor: Cole, Curtis, Ringoes, NJ 08551 (US); Ganopolsky, Irina, Lawrenceville, NJ 08648 (US); Aleles, Margaret, Gladstone, NJ 07931 (US); Clark, Patti, New Hope, PA 18938 (US); Lukenbach, Elvin, Flemington, NJ 08822 (US)
(74) Representative: Mercer, Christopher Paul

(57) **Abstract**

The invention relates to a method for ameliorating redness or inflammation of mammalian skin comprising topically applying a composition comprising an effective amount of a compound selected from an alkanolamine, tyrosine or mixtures thereof and a cosmetically acceptable carrier. The alkanolamine has the following general formula: 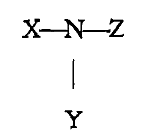 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group. In another embodiment, the invention relates to a method for ameliorating the irritating effects of a skin irritating composition comprising adding to said composition an effective amount of a compound.

## Description

### 1. Field of the Invention

This invention relates to compositions and methods for reducing inflammation and redness of mammalian skin, More particularly, it relates to compositions containing at least one compound selected from an alkanolamine and/or tyrosine and their application to mammalian skin. The compositions can be applied to skin to effect a reduction in inflammation or erythema caused by inherent skin conditions such as acne or rosacea or by irritations experienced by the skin.

### 2. Background of the Invention

Human beings have long sought products that can ameliorate irritation and redness in the skin. Such redness and irritation may be caused by inherent disease conditions such as acne, rosacea, atopic dermatitis and other disease states, but by external irritation. Substances applied to the skin and those to which the skin is exposed in daily life can cause the skin to respond by becoming red and irritated. The skin's blotchy appearance when such conditions exist may be the source of discomfort and, possibly, acute embarrassment, particularly among those stricken with disease states.

Heretofore, redness and irritation of the skin have often been treated with topical steroids. However, prolonged use of topical steroids can cause the skin to atrophy and may not be tolerated well over longer time periods.

Thus, it is an object of this invention to provide topical compositions that can be used to ameliorate redness and/or inflammation of mammalian skin.

It is another object of this invention is to provide topical compositions to ameliorate redness and inflammation that are well-tolerated by the skin.

Yet another object of this invention is to provide a method of ameliorating redness or inflammation of the skin using a topically-applied composition.

Still another object of this invention is to provide a method of ameliorating redness or inflammation quickly in order to relieve symptoms.

Yet another object of this invention is to provide a composition capable of relieving redness and inflammation which is safe for continued use over a long time period.

### Summary of the Invention

It has been discovered that compositions containing at least one compound selected from an alkanolamine and/or tyrosine alleviate redness as well as irritation on mammalian skin. Indeed, such compositions have been found to reduce redness caused by acne lesions within one hour of application. They have also been found to reduce the appearance of redness associated with rosacea.

Accordingly, in one embodiment, the invention relates to a method for ameliorating redness or inflammation of mammalian skin comprising topically applying a composition comprising an effective amount of at least one compound selected from an alkanolamine, tyrosine, or mixtures thereof and a cosmetically acceptable carrier. The alkanolamine has the following general formula: 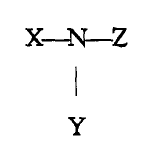 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group.

It has also been discovered that the compounds described above are effective in reducing the skin irritation caused by application of an irritating active ingredient Accordingly, in another embodiment, the invention relates to a method for ameliorating the irritating effects of a skin irritating composition comprising adding to said composition an effective amount of at least one compound as described above.

### Brief Description of the Drawings

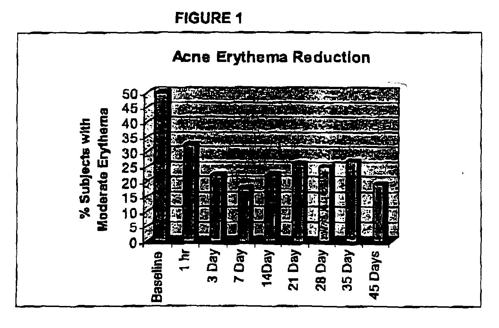

Figure 1 is a bar graph showing the percent of subjects with acne related redness over a 45 day evaluation period in accordance with the claimed method.

### Detailed Description of the Preferred Embodiments

As discussed above, the invention relates to a method for ameliorating redness or inflammation of mammalian skin comprising topically applying a composition comprising an effective amount of at least one compound selected from an alkanolamine, tyrosine, or mixtures thereof and a cosmetically acceptable carrier. The alkanolamine has the following general formula: 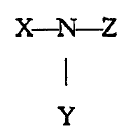 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group.

In a preferred embodiment the alkanolamine is selected from the group consisting of ethylaminoethanol, methylaminoethanol, dimethylaminoethanolamine, isopropanolamine, triethanolamine, isopropanoldimethylamine, ethylethanolamine, 2-butanolamine, choline and serine. More preferably, the alkanolamine is dimethylaminoethanol (DMAE).

The compositions used in the methods according to the invention preferably contain from about 0.1 about 10% by weight of the at least one compound, more preferably, from about 0.1 to about 5% and, most preferably, from about 1 to about 3% by weight. In a particularly preferred embodiment, the compositions used in the methods according to the invention, comprise a mixture of about 1 to about 5% by weight DMAE and from about 1 to about 5% by weight of tyrosine.

In a preferred embodiment, the compositions used in the methods of the invention contain a pH buffering agent. Preferably, the amount of buffering agent should be that which would result in compositions having a pH ranging from about 4.5 to about 8.5, more preferably from about 5.5 to about 8.5, most preferably from about 6.5 to about 8.0. The buffering agent can be any of the known buffering agents commonly found in cosmetic compositions provided that they are physically and chemically stable with the other ingredients of the composition. Suitable buffering agents include organic acids such as (but not intended to be restricted to) citric acid, malic acid, and glycolic acid.

The compositions of this invention should be in the form of topical products that can be applied externally to the skin and can be prepared in accordance with conventional techniques known to those of ordinary skill in the art. The carrier may take a variety of physical forms such as, for example, creams, dressings, gels, lotions, ointments or liquids. The carrier may take a variety of physical forms such as, for example, creams, dressings, gels, lotions, ointments or liquids. Preferably, the carrier should be a gel or moisturizing lotion, or a cooling solution. One could also utilize this in a convenient spray applicator.

Typical carriers include lotions containing water and/or alcohols and emollients such as hydrocarbon oils and waxes, silicone oils, hyaluronic acid, vegetable, animal or marine fats or oils, glyceride derivatives, fatty acids or fatty acid esters or alcohols or alcohol ethers, lanolin and derivatives, polyhydric alcohols or esters, wax esters, sterols, phospholipids and the like, and generally also emulsifiers (nonionic, cationic or anionic), although some of the emollients inherently possess emulsifying properties. These same general ingredients can be formulated into a cream rather than a lotion, or into gels, or into solid sticks by utilization of different proportions of the ingredients and/or by inclusion of thickening agents such as gums or other forms of hydrophillic colloids. Such compositions are referred to herein as cosmetically acceptable carriers. Preferably, the carrier should be a gel base formula without lipid materials that would exxacerbate the oiliness of acne prone skin. However, a moisturizer emulsion base may be preferred by individuals that have particularly dry yet skin still suffer from acne lesions.

The topical compositions according to the invention can comprise additional ingredients commonly found in skin care compositions, such as for example, emollients, skin conditioning agents, emulsifying agents, humectants, preservatives, antioxidants, perfumes, chelating agents, etc., provided that they are physically and chemically compatible with the other components of the composition. It is also envisioned that this invention could be combined with other agents such as topical anesthetics (such as benzocaine or other caine type molecules) or even mild steroids such as hydrocortisone for enhanced antiinflammatory activity]. Noteably useful is the incorporation of vitamin A and vitamin A derivatives, including but not restricted to retinoids, such as, retinol, retinyl palmitate, retinoic acid, retinal, and retinyl propionate.

Examples of suitable preservatives for use in the compositions of the invention include the C₁-C₄ alkyl parabens and phenoxyethanol. Generally, the preservative is present in an amount ranging from about 0.5 to about 2.0, preferably about 1.0 to about 1.5, weight percent based on the total composition. In a preferred embodiment, the preservative is mixture of from about 0.2 to about 0.5 weight percent methylparaben, from about 0.2 to about 5.0 weight percent propylparaben and from about 0.05 to about 0.10 weight percent butylparaben. A particularly preferred commercially available preservative that may be used in the skin care composition according to this invention is PHENONIP TM which is a practically colorless, viscous, liquid mixture of phenoxyethanol, methylparaben, ethylparaben, propylparaben, and butylparaben available from Nipa Laboratories, Inc., Wilmington, Del.

Preferably, antioxidant should be present in the compositions according to the invention. Suitable antioxidants include butylated hydroxy toluene (BHT), ascorbyl palmitate, butylated hydroanisole (BHA), phenyl-α-naphthylamine, hydroquinone, propyl gallate, nordihydroquiaretic acid, vitamin E or derivatives of vitamin E, vitamin C and derivatives thereof, calcium pantothenic, green tea extracts and mixed polyphenosls, and mixtures thereof. Of the above, the most preferred antioxidant is BHT. Preferably, the antioxidant present in the composition at from about .02 to about .05% by weight, most preferably from about .02 to about 0.10% by weight.

Emollients which can be included in the compositions of the invention function by their ability to remain on the skin surface or in the stratum corneum to act as lubricants, to reduce flaking, and to improve the skin appearance. Typical emollients include fatty esters, fatty alcohols, mineral oil, polyether siloxane copolymers and the like. Examples of suitable emollients include, but are not limited to, polypropylene glycol ("PPG")-15 stearyl ether, PPG-10 cetyl ether, steareth-10, oleth-8, PPG-4 lauryl ether, vitamin E acetate, PEG-7 glyceryl cocoate, lanolin, cetyl alcohol, octyl hydroxystearare, dimethicone, and combinations thereof. Cetyl alcohol, octyl hydroxystearate, dimethicone, and combinations thereof are preferred. When utilized, the emollient can be present in an amount from about 0.01 to about 5, preferably from about 1 to about 4% by weight of the composition.

Polyhydric alcohols can be utilized as humectants in the compositions of the invention. The humectants aid in increasing the effectiveness of the emollient, reduce scaling, stimulate removal of built-up scale and improve skin feel. Suitable polyhydric alcohols include, but are not limited to, glycerol (also known as glycerin), polyalkylene glycols, alkylene polyols and their derivatives, including butylene glycol, propylene glycol, dipropylene glycol, polypropylene glycol, polyethylene glycol and derivatives thereof, sorbitol, hydroxypropyl sorbitol, hexylene glycol, 1,3-dibutylene glycol, 1,2,6,-hexanetriol, ethoxylated glycerol, propoxylated glycerol and mixtures thereof. Glycerin is preferred. When utilized, the humectant is present in an amount from about 0.1 to about 5, preferably from about 1 to about 3 percent by weight, based on the total weight of the composition.

The compositions according to the invention preferably contain an effective stabilizing amount of an emulsifier. Preferably, the emulsifier is present at from about 1.0 to about 10.0, more preferably from about 3.0 to about 6.0, weight percent, based on the total composition. Any emulsifier that is compatible with the components of the composition can be employed. Suitable emulsifiers include stearic acid, cetyl alcohol, stearyl alcohol, steareth 2, steareth 20, Acrylates/C10-30 alkyl Acrylate Crosspolymer. Particularly preferred is PEMULEN TR-1 (CTFA Designation: Acrylates/10-30 Alkyl Acrylate Crosspolymer).

Any fragrance may be added to the compositions of the invention for aesthetic purposes. Suitable fragrances include, but are not limited to, eucalyptus oil, camphor synthetic, peppermint oil, dove oil, lavender, chamomile and the like. When utilized, fragrances are present in an amount from about 0.05 to about 0.5, preferably from about 0.1 to about 0.3 percent by weight, based on the total weight of the composition. In certain aspects of this invention, the compositions should include a chelating agent. Chelating agents which are useful in the compositions of the present invention include ethylenediamine tetra acetic acid (EDTA) and derivatives and salts thereof, dihydroxyethyl glycine, tartaric acid, and mixtures thereof. The chelating agents should be utilized in a stabilizing effective amount and may range from about 0.01 to about 2% based on the weight of the total composition, preferably from about 0.05 to about 1%. Most preferably, the chelating agent should be EDTA.

Generally, the composition is topically applied to the affected skin areas in a predetermined or as-needed regimen to bring about improvement, it generally being the case that gradual improvement is noted with each successive application. Insofar as has been determined based upon clinical studies to date, no adverse side effects are encountered. The methods according to the invention can be used to treat a variety of skin conditions which result in inflammation or erythema. For example, inflammation or erythema can result from external causes such as sun or wind burn or irritating soaps or cleansers. It is also known that inflammation and erythema can be caused from inherent conditions such as rosacea, atopic dermatitis, or allergic skin reactions. The method according to the invention can be used to treat inflammation and/or erythema caused by both external and inherent conditions.

It has also been discovered that the compounds described above are effective in reducing the skin irritation caused by application of an irritating active ingredient. Accordingly, in another embodiment, the invention relates to a method for ameliorating the irritating effects of a skin irritating composition comprising adding to said composition an effective amount of at least one compound selected from the group consisting of an alkanolamine; tyrosine; or a mixture thereof as described above. Indeed, as shown by the Examples below, it has been discovered that the irritating effects of compositions containing an irritating ingredient, such as for example, benzoyl peroxide, alpha-hydroxyacids and derivatives thereof, salicylic acid, surfactants, soaps, natural plant extracts, sunscreen actives, urea, preservatives, and retinoids, such as, retinol, retinyl palmitate, retinoic acid, retinal, and retinyl propionate, can be dramatically reduced upon the incorporation of at least one compound selected from an alkanolamine, tyrosine, or mixtures thereof as described above. One can envision a wide variety of therapeutic agents that are beneficial for the skin yet can cause short term inflammation that would benefit from the current method for ameliorating redness and/or inflammation.

The advantages of the invention and specific embodiments of the skin care compositions prepared in accordance with the present invention are illustrated by the following examples. It will be understood, however, that the invention is not confined to the specific limitations set forth in the individual examples, but rather defined within the scope of the appended claims.

The following materials were used in the Examples: BIOSIL Basics SPQ:
silicone quaternium-13 slip/conditioning agent commercially available from Biosil Technologies, Inc.
BRIJ 72: steareth 2 emulsifier commercially available from Uniqema.
BRIJ 721: steareth 20 emulsifier commercially available from Uniqema. CRODESTA SL-40: sucrose cocate skin conditioning agent/emollient commercially available from Croda Oleochemicals.
DIMETHICONE 47V-100: dimethicone 100 centistokes emollient commercially available form Rhodia.
EMERESSENCE 1160: phenoxyethanol commercially available from Cognis. FINSOLV TN: C₁₂-C₁₅ alkyl benzoate solubilizing agent commercially available from Finetex.
GERMABEN IIE preservative available from Sutton.
GLYCEROX 767: PEG-6 capric/caprylic glycerides skin conditioning agent/emollient commercially available from Croda.
GLYPURE: 70% aqueous solution of glycolic acid commercially available from DuPont
ORDENONE: odor masking fragrance commercially available from Bell Aire. PHENONIP: mixture of phenoxyethanol, methylparaben, echylparaben, propylparaben, and butylparaben commercially available from Nipa Laboratories, Inc.
SP-10: nylon-12 slip/detactifying agent commercially available from Kobo Products
STABILIEZE QM: PVM/MA decadiene crosspolymer thickener commercially available from ISP Technologies.
TWEEN 80: surfactant from Uniqema
WICKENOL 171: octyl hydroxystearate emollient commercially available from Alzo Inc.
WINDSOR TALC 66: talc detactifying/slip agent commercially available from Luzenac/Royston.

### Example 1

The following formula was made in accordance with the teachings of this invention. The oil phase, water phase and tyrosine premix were formed separately. The tyrosine premix was prepared as follows: deionized water, DMAE, and tyrosine were added to a closed container and placed in a heated (50-55°C) water bath. The mixture was heated to about 50 to about 55°C. The mixture was held at that temperature with mixing until the water and oil phases were combined and cooled to about 45°C as described below.

The following oil phase ingredients (FINSOLV TN, WICKENOL 171, Dimethicone 47V-100, BRIJ 72, CETAL, BRIJ 721, and BHT) were blended together in a kettle and heated to about 60°C with agitation. Once the mixture was homogeneous PEMULEN was added with agitation until homogenous. The temperature was held at about 78-80°C until the water phase was prepared and ready for addition.

The water phase was prepared as follows: deionized water was added to a kettle and the kettle slowly heated to about 70 to about 75°C. During the heating process disodium EDTA, glycerin, panthenol (preheated until easy flowing liquid) were added. At about 78 to about 80°C propylparaben, methylparaben and phenoxyethanol were added. The mixture was held at about 70 to about 75°C for about 3 to about 5 minutes, *i.e*., until a uniform mixture was obtained.

When both phases were at a temperature of about 78 to about 80°C and homogenous, the oil phase was added to the water phase. The mixture was held at about 78 to about 80°C for about 10 to 15 minutes, *i.e*., until a smooth, non-grainy dispersion was apparent. Heating was discontinued and when the temperature reached below 45°C the DMAE/Tyrosine premix followed by the buffer pre-mix were added and mixed well. The mixture was homogenized at 40% for about 3-4 minutes with a rotor-stator homogenizer.

| **INGREDIENT:** | **WEIGHT PERCENT**: |
|---|---|
| *Water Phase:* | |
| Deionized water | 62.69 |
| Disodium EDTA | 0.10 |
| Glycerin | 3.00 |
| Panthenol | 0.50 |
| EMERESSENCE 1160 | 0.73 |
| Methylparaben | 0.35 |
| Propylparaben | 0.17 |

| *Oil Phase:* | |
|---|---|
| FINSOLV TN | 4.00 |
| WICKENOL 171 | 1.00 |
| Dimethicone 47V-100 | 1.00 |
| BRIG 72 | 0.60 |
| Cetyl Alchol | 2.50 |
| BRIJ 721 | 0.90 |
| BHT | 0.10 |
| PEMULEN TR1 | 0.50 |

| *DMAE/Tyrosine Premix:* | |
|---|---|
| L-tyrosine | 0.50 |
| Deionized water | 15.00 |
| DMAE | 3.00 |

| *Buffer Premix:* | |
|---|---|
| GLYPURE 70 | 1.2 |
| Malic acid | 0.84 |
| Deionized water | 1.32 |

The composition of Example 1 was applied to 29 females having mild acne at the start of the study for a 45 day period. The females had the following demographics:

| No. of Subjects | Acne | Mean Age | Gender | Mean Weight |
|---|---|---|---|---|
| 29 | Mild* | 30 | Female | 157 lbs. |

| | | | | |
|---|---|---|---|---|
| *A subject with "mild acne" had greater than 10 non-inflammatory and inflammatory lesions at the start of the study, i.e., "baseline". | | | | |

Of the 29 subjects with mild acne, about 49% had moderate erythema. The level of inflammation redness and inflammation exhibited by the subjects during the test period was evaluated by a dermatologist. The results are set forth in Figure 1. As can be seen from Figure 1, a statistically significant decrease in redness at all observations was seen when compared to the baseline.

### Comparative Example 2

Deionized water was added to a kettle and heated to about 78 to about 80°C. At about 78 to about 82°C, STABILEZE QM was added using a propeller mixer. The mixture was held at about 78 to about 82°C until dear. Heating was discontinued and when the mixture was at about 75°C, disodium EDTA, CRODESTA SL-40, GLYCEROX 767, and hexylene glycol were added. At room temperature, the urea/water premix was added to the mixture. The pH of the mixture was adjusted to about 6.5 to about 7.0 with potassium hydroxide 10% solution. The remaining ingredients were added with mixing in the following order. SD-10, talc, BIOSIL BASICS SPQ, PHENONIP, and deionized water. The mixture was homogenized at 40% for about 2 minutes with a rotor-stator homogenizer.

| **INGREDIENT:** | **WEIGHT PERCENT:** |
|---|---|
| Deionized water | 70.85 |
| STABILEZE QM | 1.50 |
| Disodium EDTA | 0.10 |
| CRODESTA SL-40 | 0.75 |
| GLYCEROX 767 | 0.75 |
| Hexylene glycol | 1.00 |
| KOH, 10% solution | 6.55 |

| *Urea Premix:* | |
|---|---|
| Deionized water | 10.00 |
| Urea | 5.00 |

| *Post Addition:* | |
|---|---|
| Windsor Talc 66 | 0.50 |
| SP-10 | 1.00 |
| BIOSIL Basics SPQ | 1.00 |
| PHENONIP | 1.00 |

### Comparative Example 3

Deionized water was added to a kettle, argon was bubbled though for 10 minutes to purge oxygen out. Then, the water was heated to about 78 to about 80°C. Under argon blanket, at about 78 to about 82°C, STABILEZE QM was added using a propeller mixer. The mixture was held at about 78 to about 82°C until clear. Heating was discontinued and when the mixture was at about 75°C, disodium EDTA, CRODESTA SL-40, GLYCEROX 767, and hexylene glycol were added. At room temperature, the urea premix was added to the mixture and mixed well. The pH of the mixture was adjusted to about 6.5 to about 7.0 with potassium hydroxide 10% solution. Under yellow lights and with argon purging the remaining ingredients were added with mixing in the following order. Tween 80, Retinol, SD-10, talc, BIOSIL BASICS SPQ, PHENONIP, and deionized water. The mixture was homogenized at 40% for about 2 minutes with a rotor-stator homogenizer.

| **INGREDIENT** | **WEIGHT PERCENT:** |
|---|---|
| Deionized water | |
| STABILEZE QM | 1.50 |
| Disodium EDTA | 0.10 |
| CRODESTA SL-40 | 0.75 |
| GLYCEROX 767 | 0.75 |
| Hexylene glycol | 1.00 |
| KOH, 10% solution | |

| *Urea Premix:* | |
|---|---|
| Deionized water | 10.00 |
| Urea | 5.00 |

| *Post Addition:* | |
|---|---|
| TWEEN 80 | 0.15 |
| Retinol | 0.1 |
| Windsor Talc 66 | 0.50 |
| SP-10 | 1.00 |
| BIOSIL Basics SPQ | 1.00 |
| PHENONIP | 1.00 |

### Example 4

Deionized water was added to a kettle and heated to about 78 to about 80°C. At about 78 to about 82°C, STABILEZE QMwas added using a propeller mixer. The mixture was held at about 78 to about 82°C until clear. Heating was discontinued and when the mixture was at about 75°C, disodium EDTA, CRODESTA SL-40, GLYCEROX 767, and hexylene glycol were added. At room temperature, the DMAE premix was added to the mixture to neutralize the STABILEZE followed by the tyrosine premix and mixed well. The pH of the mixture was adjusted to about 6.5 to about 7.0 with glycolic acid. The remaining ingredients were added with mixing in the following order: SD-10, talc, BIOSIL BASICS SPQ, PHENONIP, and deionized water. The mixture was homogenized at 40% for about 2 minutes with a rotor-stator homogenizer.

| **INGREDIENT:** | **WEIGHT PERCENT:** |
|---|---|
| Deionized water | 63.8 |
| STABILEZE QM | 1.50 |
| Disodium EDTA | 0.10 |
| CRODESTA SL-40 | 0.75 |
| GLYCEROX 767 | 0.75 |
| Hexylene glycol | 1.00 |

| *DMAE Premix:* | |
|---|---|
| DMAE | 3.00 |
| Deionized water | 3.00 |

| *Tyrosine/Urea Premix:* | |
|---|---|
| Deionized water | 10.00 |
| Urea | 5.00 |
| L-tyrosine | 5.00 |

| *Post Addition:* | |
|---|---|
| GLYPURE | 2.61 |
| Windsor Talc 66 | 0.50 |
| SP-10 | 1.00 |
| BIOSIL Basics SPQ | 1.00 |
| PHENONIP | 1.00 |

### Comparative Example 5

Warer, sodium hyaluronare, urea, glycosaminoglycans, xantham gum, EDTA-Na2, and sorbitol were dissolved to form Phase 1. This phase was heated to 65C. Butyleneglycol dicaprylate/dicaprate, cyclomethicone, ascorbyl palmitate, polysorbate 20, oleic acid, vitamin E acetate, and cetearyl alcohol 50/50 were combined to form Phase 2 and were heated to 62C. In the presence of a homogenizer, Phase 1 was added to Phase 2 with continued homogenization for 10 minutes. Titanium dioxide and GERMABEN IIE were then added with additional water. The emulsion was homogenized for an additional 20 minutes. The emulsion was cooled rapidly using sweep mixing to 50C. The emulsion was additionally cooled to 45C at which time ORDENONE was added.

| INGREDIENT | WEIGHT PERCENT |
|---|---|
| Water | 64.90 |
| Sodium hyaluronate | 11.00 |
| Ascorbyl palmitate | 7.00 |
| L-tyrosine | NONE |
| Urea | 5.00 |
| Butyleneglycol dicaprylate/dicaprate | 4.00 |
| Cyclomethicone | 3.00 |
| Glycosoamineglycans | 2.00 |
| DMAE | NONE |
| Glycolic acid (70%) | NONE |
| Polysorbate-20 | 0.60 |
| Oleic acid | 0.60 |
| Sorbitol 70% | 0.50 |
| Germaben IIE | 0.50 |
| Ordenone | 0.20 |
| Sodium Metabisulfite | 0.20 |
| Disodium EDTA | 0.10 |
| Vitamin E Acetate | 0.10 |
| Cetearyl alcohol 50/50 | 0.10 |
| TiO2 | 0.10 |
| Xanthan Gum | 0.10 |

### Example 6

The product was processed by pre-dissolving the glycolic acid with the water, sodium hyaluronate, urea, glycosaminoglycans, xantham gum, EDTA-Na2, and sorbitol to form Phase 1. This phase was heated to 65C. Butyleneglycol dicaprylate/dicaprate, cydomethicone, ascorbyl palmitare, polysorbate 20, oleic acid, vitamin E acetate, and cetearyl alcohol 50/50 were combined to form Phase 2 and were heated to 62C. In the presence of a homogenizer, Phase 1 was added to Phase 2 with continued homogenization for 10 minutes. L-tyrosine, titanium dioxide, and germaben IIE were then added with additional water. The emulsion was homogenized for an additional 20 minutes. The emulsion was cooled rapidly using sweep mixing to 45C at which time the ORDENONE was added.

| INGREDIENT | WEIGHT PERCENT |
|---|---|
| Water | 58.97 |
| Sodium hyaluronate | 11.00 |
| Ascorbyl palmitate | 7.00 |
| L-tyrosine | 5.00 |
| Urea | 5.00 |
| Butyleneglycol dicaprylate/dicaprate | 4.00 |
| Cyclomethicone | 3.00 |
| Glycosoamineglycans | 2.00 |
| DMAE | NONE |
| Glycolic acid (70%) | 1.00 |
| Polysorbate-20 | 0.60 |
| Oleic acid | 0.60 |
| Sorbitol 70% | 0.50 |
| Germaben IIE | 0.50 |
| Ordenone | 0.20 |
| Sodium Metabisulfite | 0.20 |
| Disodium EDTA | 0.10 |
| Vitamin E Acetate | 0.10 |
| Cetearyl alcohol 50/50 | 0.10 |
| TiO2 | 0.10 |
| Xanthan Gum | 0.03 |

### Example 7

The product was processed by pre-dissolving the glycolic acid with the water, sodium hyaluronate, urea, glycosaminoglycans, xantham gum, EDTA-Na2, and sorbitol to form Phase 1. This phase was heated to 65C. Butyleneglycol dicaprylate/dicaprate, cyclomethicone, ascorbyl palmitare, polysorbate 20, oleic acid, vitamin E acetate, and cetearyl alcohol 50/50 were combined to form Phase 2 and were heated to 62C. In the presence of a homogenizer, Phase 1 was added to Phase 2 with continued homogenization for 10 minutes. Titanium dioxide, and germaben IIE were then added with additional water. The emulsion was homogenized for an additional 20 minutes. The emulsion was cooled rapidly using sweep mixing to 50C, and the dimethylaminoethanol was added with equal part of water. The emulsion was additionally cooled to 45C at which time the ordenone was added.

| INGREDIENT | WEIGHT PERCENT |
|---|---|
| Water | 60.97 |
| Sodium hyaluronate | 11.00 |
| Ascorbyl palmitate | 7.00 |
| L-tyrosine | NONE |
| Urea | 5.00 |
| Butyleneglycol dicaprylate/dicaprate | 4.00 |
| Cyclomethicone | 3.00 |
| Glycosoamineglycans | 2.00 |
| DMAE | 3.00 |
| Glycolic acid (70%) | 1.00 |
| Polysorbate-20 | 0.60 |
| Oleic acid | 0.60 |
| Sorbitol 70% | 0.50 |
| Germaben IIE | 0.50 |
| Ordenone | 0.20 |
| Sodium Metabisulfite | 0.20 |
| Disodium EDTA | 0.10 |
| Vitamin E Acetate | 0.10 |
| Cetearyl alcohol 50/50 | 0.10 |
| TiO2 | 0.10 |
| Xanthan Gum | 0.03 |

### Example 8

The product was processed by pre-dissolving the glycolic acid with the water, sodium hyaluronate, urea, glycosaminoglycans, xantham gum, EDTA-Na2, and sorbitol to form Phase 1. This phase was heated to 65C. Butyleneglycol dicaprylate/dicaprate, cyclomethicone, ascorbyl palmitare, polysorbate 20, oleic acid, vitamin E acetate, and cetearyl alcohol 50/50 were combined to form Phase 2 and were heated to 62C. In the presence of a homogenizer, Phase 1 was added to Phase 2 with continued homogenization for 10 minutes. L-tyrosine, titanium dioxide, and germaben IIE were then added with additional water. The emulsion was homogenized for an additional 20 minutes. The emulsion was cooled rapidly using sweep mixing to 50C, and the dimethylaminoethanol was added with equal part of water. The emulsion was additionally cooled to 45C at which time the ordenone was added.

| INGREDIENT | WEIGHT PERCENT |
|---|---|
| Water | 55.97 |
| Sodium hyaluronate | 11.00 |
| Ascorbyl palmitate | 7.00 |
| L-tyrosine | 5.00 |
| Urea | 5.00 |
| Butyleneglycol dicaprylate/dicaprate | 4.00 |
| Cyclomethicone | 3.00 |
| Glycosoamineglycans | 2.00 |
| DMAE | 3.00 |
| Glycolic acid (70%) | 1.00 |
| Polysorbate-20 | 0.60 |
| Oleic acid | 0.60 |
| Sorbitol 70% | 0.50 |
| Germaben IIE | 0.50 |
| Ordenone | 0.20 |
| Sodium Metabisulfite | 0.20 |
| Disodium EDTA | 0.10 |
| Vitamin E Acetate | 0.10 |
| Cetearyl alcohol 50/50 | 0.10 |
| TiO2 | 0.10 |
| Xanthan Gum | 0.03 |

In a second series of studies, inflammation was observed to be reduced when products were formulated in accordance with the invention compared with formulae without these elements. Specifically, the compositions of Examples 2-8 were tested in a modified RIPT (Draize) text as set forth in U.S. patent application Ser. No. 08/414,975. The compositions were applied repeatedly to the skin of approximately 200 volunteers under semi-occlusive patches over a period of three weeks. At each application, the level of inflammation under each test patch was rated on a scale of 0 (no visible erytherna or inflammation) to 4 (intense erythema with edema and erosion). The irritation scores for each product, for *each* observation, for each subject were summed to yield a composite irritation score for each of the test formulae. The results are set forth below.

| Example | Irritation Score |
|---|---|
| 2 | 17.5 |
| 3 | 365 |
| 4 | 281 |
| 5 | 128.5 |
| 6 | 61.5 |
| 7 | 18.5 |
| 8 | 15.5 |

The results show that the addition of retinol (Example 3) to the gel base (Comparative Example 2) there was a large increase in the irritation scores from 17.5 to 365. Addition of 3% dimethylaminoethanol (DMAE) and 5% tyrosine to the same base with 0.15% retinol (Example 4) showed an anti-inflammatory effect on the irritation reducing the irritation to 281 Similarly a moderately irritating moisturizer base (Comparative Example 5) had an irritation score of 128.5. The addition of 5% tyrosine (Example 6) or 3% DMAE (Example 7) reduced the irritation score dramatically to 61.5 and 18.5 respectively. Further, the combination of tyrosine and DMAE (Example 8) demonstrated an even more dramatically lower irritation compared with the irritating placebo. These demonstrate the unexpected result that either tyrosine or DMAE separately, or in combination can reduce the irritation of irritating skin compositions or compositions that have irritating ingredients.

### Example 9

The following composition was prepared in accordance with the procedure described in Example 4.

| Ingredient | Weight Percent |
|---|---|
| | |
| DI Water | 90.90 |
| PVM/MA Decadiene Crosspolymer | 1.50 |
| Disodium EDTA | 0.10 |
| Sucrose Cocoate | 0.75 |
| PEG-6 Capric/Caprylic Glycerides | 0.75 |
| Hexylene Glycol | 1.00 |
| | |
| KOH 10% solution | 1.00 |
| | |
| Talc | 0.50 |
| Nylon 12 | 1.00 |
| Silicone Quaternium-13 | 1.00 |
| Ethanol | 0.50 |
| Phenoxyethanol, Methylparaben, Butylparaben, Ethylparaben, and Propylparaben | 1.00 |

The composition of Example 9 was applied to a subject suffering from rosacea. After four days of daily use of the formula, there was significant visual reduction in the redness, blotchiness, and uneven surface texture associated with the rosacea condition.

## Claims

1. A composition comprising
(a) an effective amount of at least one compound selected from the group consisting of an alkanolamine, tyrosine or a mixture thereof; and
(b) a cosmetically acceptable carrier,
wherein said alkanolamine has the following general formula: 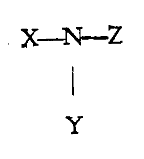 wherein X, Y and Z are selected from the group consisting of hydrogen, C₁-C₃ alkyl group, and C₂-C₄ alkanol group, wherein at least one of X, Y or Z is a C₂-C₄ alkanol group bearing at least one hydroxyl group and optionally at least one carboxyl group.

2. The composition of claim 1, wherein said alkanolamine is ethylaminoethanol, methylaminoethanol, dimethylaminoethanolamine, isopropanolamine, triethanolamine, isopropanoldimethylamine, ethylethanolamine, 2-butanolamine, choline or serine.

3. The composition of claim 2, wherein said alkanolamine is dimethylaminoethanol.

4. The composition of any one of claims 1 to 3, wherein said at least one compound is present in an amount of from 0.1 to 10% by weight of the composition.

5. The composition of claim 4, wherein said composition comprises a mixture of from 1 to 5% by weight of alkanolamine and from 1 to 5% by weight of tyrosine.

6. The composition of any one of claims 1 to 5, which further comprises a skin irritating ingredient.

7. The composition of claim 6, wherein the irritating ingredient is a retinoid, benzyol peroxide, an alpha-hydroxyacid or a derivative thereof, salicylic acid, a surfactant, a soap, a natural plant extract, a sunscreen active, urea or a preservative.

8. The composition of any one of claims 1 to 7, for use in ameliorating redness or inflammation of mammalian skin, for instance by application to inflamed skin, sun burned skin, wind burned skin, skin that is red or inflamed due to contact with irritating soaps or cleaners, or skin that is red or inflamed due to rosacea, atopic dermatitis or an allergic skin reaction.

9. A method for ameliorating the irritating effects of a skin irritating composition comprising adding to said composition an effective amount of a compound selected from the group consisting of an alkanolamine, tyrosine or a mixture thereof; wherein said alkanolamine has the following general formula: 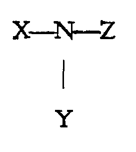 as defined in claim 1.

10. The method of claim 9, wherein said alkanolamine is as defined in claim 2 or claim 3.

11. The method of claim 9 or 10, wherein said at least one compound is present in an amount of from 0.1 to 10% by weight of the composition.

12. The method of any one of claims 9 to 11, wherein said composition comprises a mixture of from 1 to 5% by weight of alkanolamine and from 1 to 5% by weight of tyrosine.

13. The method of any one of claims 9 to 12, wherein the skin irritating composition comprises an irritant which is a retinoid, benzyol peroxide, an alpha-hydroxyacid or a derivative thereof, salicyclic acid, a surfactant, a soap, a natural plant extract, a sunscreen active, urea or preservative.
